# Europäisches Patentamt
# European Patent Office
# Office européen des brevets

(11) Numéro de publication: **0 299 873 B1**

(12) **FASCICULE DE BREVET EUROPEEN**

(19)

(45) Date de publication de fascicule du brevet: **17.02.93**

(21) Numéro de dépôt: **88401835.9**

(22) Date de dépôt: **13.07.88**

(51) Int. Cl.⁵: **C07C 45/49**, C07C 47/06, C07C 47/02

(54) **Procédé de préparation d'aldéhydes.**

(30) Priorité: **16.07.87 FR 8710044**

(43) Date de publication de la demande:
**18.01.89 Bulletin 89/03**

(45) Mention de la délivrance du brevet:
**17.02.93 Bulletin 93/07**

(84) Etats contractants désignés:
**AT BE CH DE ES FR GB GR IT LI LU NL SE**

(56) Documents cités:
**US-A- 4 511 741**
**US-A- 4 513 151**

**CHEMICAL ABSTRACTS, vol. 101, 1984, page 698, résumé no. 191141h, Columbus, Ohio, US; & JP-A-59 104 329 (MITSUBISHI GAS CHEMICAL CO., INC.) 16-06-1984**

(73) Titulaire: **SOLLAC**
**Immeuble Elysées-La Défense 29, le Parvis**
**F-92072 Puteaux(FR)**

(72) Inventeur: **Cordier, Jean**
**48 rue Henri Barbusse**
**F 59880 Saint Saulve(FR)**
Inventeur: **Dussart, Bernard**

**4 rue du Contade**
**F-67300 Schiltigheim(FR)**
Inventeur: **Castanet, Yves**
**29 allée de la Comédie**
**F-59650 Villeneuve d'Asq(FR)**
Inventeur: **Melloul, Serge**
**12 Bis rue d'Esquermes**
**F-59000 Lille(FR)**
Inventeur: **Petit, Francis**
**13 allée de la Clairière**
**F-59650 Villeneuve d'Asq(FR)**
Inventeur: **Mortreux, André**
**17 rue Gambetta**
**F-59150 Hem(FR)**

(74) Mandataire: **Le Guen, Gérard et al**
**CABINET LAVOIX 2, place d'Estienne d'Orves**
**F-75441 Paris Cédex 09 (FR)**

Rank Xerox (UK) Business Services
(3.10/3.5x/3.0.1)

EP 0 299 873 B1

## Description

La présente invention concerne un procédé de préparation d'aldéhydes à partir de formiates d'alkyle et notamment d'acétaldéhyde à partir de formiate de méthyle.

L'hydrocarbonylation du méthanol en acétaldéhyde ($CH_3CHO$) est une réaction bien connue. Elle est catalysée par les métaux de transition (cobalt seul ou en synergie avec d'autres métaux...) très souvent coordinés par un ligand du type $ER_3$ ($E = P,As,Sb...$, $R = $ un radical organique). Le schéma réactionnel est le suivant

$$CH_3OH + CO + H_2 \rightarrow CH_3CHO + H_2O$$

La sélectivité en acétaldéhyde n'est jamais totale, une fraction plus ou moins importante étant hydrogénée en éthanol selon le schéma

$$CH_3CHO + H_2 \rightarrow CH_3CH_2OH$$

On a décrit dans DE-C-20 26 031 un procédé de préparation d'acide carboxylique à partir de formiate d'alkyle en présence d'un catalyseur tel que Co, Ni ou Fe et éventuellement d'un halogène ou d'un halogénure dans certaines proportions au sein de divers solvants, y compris la N-méthylpyrrolidone. Ce procédé conduit toutefois à la formation d'acide acétique. On a par ailleurs décrit un procédé semblable dans US-4 194 056 dans lequel on utilise comme catalyseur un sel de rhodium. La réaction est de préférence effectuée sans solvant et conduit à la formation d'acide acétique.

En outre, CHEMICAL ABSTRACTS, vol.101, 1984, page 698, résumé n° 191141 h, correspondant à la demande de brevet JP-A-59 104329 décrit la préparation de méthanol, d'acétaldéhyde et d'éthanol à partir de formiate de méthyle et d'un mélange d'$H_2$ et de CO.

Enfin, US-A-4,511,741 et US-A-4,513,151 décrivent un procédé de formation d'aldéhyde et d'acide carboxylique par réaction d'un ester et d'un mélange d'$H_2$ et de CO.

La présente invention vise à fournir un procédé de préparation d'aldéhydes avec une bonne sélectivité par réaction sur un formiate d'alkyle d'un gaz constitué principalement de CO.

La présente invention a ainsi pour objet un procédé de préparation d'un aldéhyde de formule

R - CHO

dans laquelle R est un groupe alkyle en $C_1$-$C_7$, caractérisé en ce que l'on fait réagir sur un formiate d'alkyle de formule

H - COOR

dans laquelle R a la signification donnée ci-dessus un gaz constitué principalement de CO, en présence de :

a) un catalyseur à base d'un métal choisi parmi Rh, Ru, Ir, leur mélange ou un mélange de Rh et/ou de Ru et/ou de Ir avec Co, Ni ou Fe, ce catalyseur étant utilisé en une proportion molaire par rapport au formiate d'alkyle de $10^{-2}$ à $10^{-4}$,

b) un promoteur choisi parmi les iodures alcalins, alcalino-terreux, d'ammonium quaternaire ou de phosphonium, et les mélanges de composés covalents d'iode et d'une phosphine de formule $PR'_3$, $R'$ étant un groupe alkyle ou aryle ou d'une amine tertiaire de formule $NR''_3$, $R''$ étant un groupe alkyle, l'iodure ou le composé covalent d'iode étant utilisés en une proportion molaire par rapport au formiate d'alkyle de $4.10^{-2}$ à $0,25.10^{-2}$,

c) un solvant comprenant un N-(alkyl en $C_1$-$C_6$) amide cyclique, l'amide cyclique étant utilisé en une proportion molaire par rapport au formiate d'alkyle d'au moins 1.

On effectue avantageusement la réaction à une température de 150 à 230°C et de préférence de 160 à 200°C et sous une pression de CO d'au moins 1 MPa et de préférence d'au moins 5 MPa. En pratique les pressions sont de préférence de 5 à 10 MPa.

Le catalyseur est de préférence à base de rhodium, de ruthénium ou d'iridium. Comme exemples de catalyseur on peut citer $RhCl_3 . 3H_2O$, $Rh_2 Cl_2 (CO)_4$, $Rh CO Cl(P Ph_3)_2$, $Rh_6(CO)_{16}$, $RhBr_3$, $RhI_3$, $RuCl_3$, $RuO_2$, $IrCl_3$.

Comme indiqué précédemment on opère en présence d'un promoteur iodé. Ce promoteur peut être un iodeur alcalin (LiI, KI, NaI) éventuellement en mélange avec du chrome hexacarbonyle ($Cr(CO)_6$., un iodure

alcalino-terreux, un iodure de phosphonium PPh$_3$ R$'''$I ou un iodure d'amonium NR$'''_4$I, R$'''$ étant un groupe alkyle en C$_1$-C$_4$.

Le promoteur iodé peut être également un mélange d'un composé covalent d'iode et de phosphine ou d'amine tertiaire. Le composé covalent d'iode peut être notamment l'iode ou un iodure d'alkyl en C$_1$-C$_4$ ou l'acide iodhydrique. La phosphine peut être une trialkylphosphine ou une triarylphosphine telle que la triphénylphosphine. La teneur molaire en phosphine ou en amine tertiaire est avantageusement au moins égale à la teneur molaire en composé covalent d'iode.

On opère en outre en présence d'un solvant comprenant un N-(alkyl en C$_1$-C$_6$) amide cyclique. Comme exemple de N-(alkyl en C$_1$-C$_6$) amide cyclique on peut citer la N-méthylpyrrolidone, la N-éthylpyrrolidone, et la N,N-diméthylimidazolidinone.

Il est à souligner que la nature du solvant utilisé est déterminante. On a constaté en effet de manière surprenante que l'on obtient des résultats très satisfaisants dans la N-méthylpyrrolidone ou d'autres N-alkyl amides cycliques mais que la réaction ne se produit pas en présence de pyrrolidone où l'on observe une décomposition du formiate en CO et méthanol. De même dans d'autres solvants usuels (toluène, DMF...) l'activité et la sélectivité en acétaldéhyde sont beaucoup plus faibles.

Le procédé selon l'invention peut être mis en oeuvre en utilisant comme gaz constitué principalement de CO de gaz d'aciérie, ce qui permet de valoriser les gaz d'acérie. Un gaz d'aciérie a par exemple la composition suivante (en % en moles) :

| | |
|---|---|
| CO | 74,5 % |
| CO$_2$ | 14,1 % |
| H$_2$ | 2,0 % |
| O$_2$ | 0,2 % |
| N$_2$ | 8,3 % |
| H$_2$S | 3 ppm |
| COS | 37 ppm |

Les exemples suivants illustrent le procédé selon l'invention.

Exemple 1 :

Dans un autoclave de 100 cm$^3$, préalablement mis sous atmosphère inerte, sont introduits 0,0295 g (1,2.10$^{-4}$ mole) de RhCl$_3$. 3H$_2$O, 0,125 g (9,3.10$^{-4}$ mole) de LiI et 5 g (87.10$^{-3}$ mole) de formiate de méthyle dissous dans 50 cm$^3$ de N-méthylpyrrolidone (NMP).

L'autoclave est chauffé à 180°C et lorsque sa température s'est stabilisée, il est pressurisé à 5 MPa de CO et mis sous agitation. La transformation du formiate de méthyle est suivie par analyse par chromatographie en phase gazeuse de prélèvements périodques, le moment de mise sous agitation étant pris comme instant initial.

Au bout de 6h, l'agitation est arrêtée et l'autoclave refroidi brusquement.

Les phases liquides et gazeuses sont analysées par chromatographie en phase gazeuse.

Les résultats sont les suivants

Phase liquide :

méthanol : 2,75%, acétalddéhyde : 42,7%, formiate de méthyle : 49%

éthanol : 1,1%, acétone : 3,4%; butanol : 0,9%

Phase gazeuse :

CO$_2$ = 11,7%, CH$_4$ = 4,2%, CO = 85%.

On a reporté dans le tableau I ci-après les résultats obtenus à l'exemple 1 ainsi que d'autres résultats (exemples 2 à 1) obtenus en utilisant le même protocole expérimental. On a également donné les résultats obtenus aux exemples comparatifs 1 et 2 où l'on a opéré en présence respectivement de toluène et de pyrrolidone, ainsi qu'aux exemples comparatifs 3 à 6 dans lesquels d'autres conditions ont été modifiées.

TABLEAU I

| Exemple n° | Conditions Expérimentales | Temps (h) | Teneur en % molaire produit | | | | | | |
|---|---|---|---|---|---|---|---|---|---|
| | | | $CH_3OH$ | $CH_3CHO$ | $HCOOCH_3$ | $C_2H_5OH$ | $CH_3COCH_3$ | AcOMe | Butanal |
| 1 | T = 180°C – $P_{CO}$ = 5MPa<br>LiI=0,93 mmole<br>HCOOCH$_3$=87 mmoles<br>RhCl$_3$,3H$_2$O=0,125 mmole<br>NMP = 50 cm$^3$ | 6 | 2,75 | 42,7 | 49 | 1,1 | 3,4 | – | 0,9 |
| 2 | T = 160°C – $P_{CO}$=12MPa<br>I$_2$ = 0,25 mmole<br>PPh$_3$=0,7 mmole<br>RhClCO(PPh$_3$)$_2$=0,125mmole<br>HCOOCH$_3$= 52 mmole<br>NMP = 50 cm$^3$ | 15 | 2,1 | 39 | 55,5 | 0,5 | 0,5 | – | 0,9 |
| 3 | T=180°C – $P_{CO}$=14MPa<br>autres conditions iden-tiques<br>n° 2 | 2<br><br><br>16 | 0,5<br><br><br>0,5 | 25;3<br><br><br>62,5 | 70<br><br><br>6,3 | 0,5<br><br><br>1,6 | 2,2<br><br><br>6,1 | –<br><br><br>– | 2,2<br><br><br>10 |
| 4 | T =180°C – $P_{CO}$=14MPa<br>PPh$_3$=1,4mmole<br>autres conditions iden-tiques<br>n° 3 | 2<br><br><br>16 | 0,2<br><br><br>0,2 | 67,4<br><br><br>77,9 | 19,1<br><br><br>0,3 | 0,3<br><br><br>0,7 | 6,1<br><br><br>3,9 | –<br><br><br>– | 4,3<br><br><br>14,5 |

EP 0 299 873 B1

TABLEAU I (suite)

| Exemple n° | Conditions Expérimentales | Temps (h) | Teneur en % molaire produit | | | | | | |
|---|---|---|---|---|---|---|---|---|---|
| | | | $CH_3OH$ | $CH_3CHO$ | $HCOOCH_3$ | $C_2H_5OH$ | $CH_3COCH_3$ | AcOMe | Butanal |
| 5 | T = 180°C – $P_{CO}$=5MPa<br>LiI=1,9mmoles<br>$RhCl_3, 3H_2O$=0,125mmole<br>$HCOOCH_3$ = 87mmoles<br>NMP = 50 $cm^3$ | 2<br><br>6 | 5,7<br><br>5,8 | 40,2<br><br>66,4 | 45<br><br>7,9 | 1,4<br><br>3,2 | 5,6<br><br>10,8 | 1,9<br><br>3,7 | –<br><br>7,7 |
| 6 | T = 140°C – $P_{CO}$=5MPa<br>autres conditions<br>identiques<br>n° 5 | 6 | 1,3 | 5,7 | 91,5 | – | 0,9 | – | 0,25 |
| 7 | T = 220°C – $P_{CO}$=5MPa<br>autres conditions<br>identiques<br>n° 5 | 1/2 | 18,8 | 37,6 | 24 | 3 | 7 | 3,5 | 1,7 |
| 8 | T = 180°C – $P_{CO}$=5MPa<br>$Rh_2Cl_2(CO)_4$=0,062mmole<br>$I_2$=0,25mmole – $PPh_3$=2mmoles<br>$HCOOCH_3$=52 mmoles<br>NMP = 50$cm^3$ | 6 | 1,43 | 50,4 | 46 | 0,2 | 1,1 | 0,08 | 0,63 |

TABLEAU I (suite)

| Exemple n° | Conditions Expérimentales | Temps (h) | Teneur en % molaire produit | | | | | | |
|---|---|---|---|---|---|---|---|---|---|
| | | | $CH_3OH$ | $CH_3CHO$ | $HCOOCH_3$ | $C_2H_5OH$ | $CH_3COCH_3$ | AcOMe | Butanal |
| 9 | $T=180°C - P_{CO}=5MPa$<br>$RhCl_3$, $3H_2O=0,125mmole$<br>$LiI=0,95mmole$<br>$PPh_3=1,65mmoles$<br>$HCOOCH_3=87mmoles$<br>$NMP = 50cm^3$ | 6 | 11,3 | 10,1 | 73 | 0,3 | 1,1 | 1,1 | 0,8 |
| 10 | $T=180°C - P_{CO}=5MPa$<br>$RuCl_3=0,125mmole$<br>$LiI =1,9mmoles$<br>$HCOOCH_3 = 87mmoles$<br>$NMP = 50cm^3$ | 3 | 10,2 | 47,3 | 30,5 | 0,3 | – | 2,5 | 2, 5 |
| Exemple comparatif 1 | $T=180°C - P_{CO}= 14MPa$<br>$RhCOCl(PPh_3)_2=0,125mmole$<br>$I_2=0,47mmole$<br>$PPh_3=3,5mmoles$<br>$HCOO CH_3=52mmoles$<br>toluène=50 $cm^3$ | 14 | 1,2 | 8,4 | 60,2 | – | – | 30,2 | – |
| Exemple comparatif 2 | $T =180°C - P_{CO}=5MPa$<br>$RhCOCl(PPh_3)_2=0,125mmole$<br>$I_2=0,47mmole-$<br>$PPh_3=3,5mmoles$<br>$HCOOCH_3 =87mmoles$<br>pyrrolidone = 50 $cm^3$ | 2 | 63 | – | 32 | – | – | 4 | |

EP 0 299 873 B1

TABLEAU I (suite)

| Exemple | Conditions Expérimentales | Temps (h) | Teneur en % molaire produit | | | | | | | |
|---|---|---|---|---|---|---|---|---|---|---|
| | | | CH$_3$OH | CH$_3$CHO | HCOOCH$_3$ | C$_2$H$_5$OH | CH$_3$COCH$_3$ | AcOMe | Butanal | CH$_3$COOH |
| 11 | T=180°C  P(CO)=5MPa<br>HI=0,1 cm$^3$ =1,25 mmole<br>PPH$_3$=1,5 mmole<br>HCOOCH$_3$=52 mmoles<br>RhCl$_3$ =0,125 mmole<br>NMP = 50 cm$^3$ | 2<br>6 | 9,5<br>9,9 | 20,5<br>59,1 | 70,0<br>29,0 | 0,4 | | | | |
| compa-ratif 3 | Identique n° 11 sans PPh$_3$ | 2 | 6,7 | 0,4 | 92,8 | | | | | |
| compa-ratif 4 | Identique n° 12 sauf HI = 2,5 mmoles | 5 | 14,7 | 0,5 | 71,5 | | | 7,7 | | 5,0 |
| compa-ratif 5 | Identique n° 12 sauf HI = 10 mmoles | 1<br>2 | 7,2<br>0,5 | 0,4<br>0,0 | 34,0<br>5,4 | 1 | | 21,0<br>7,5 | | 37,0<br>86,0 |
| compa-ratif 6 | T = 180°  P(CO) = 5 MPa<br>LiI = 3,3 mmoles<br>HCOOCH$_3$ = 0,66 mmole<br>NMP = 10 cm$^3$ | 3 | 2,1 | 1,2 | 59,0 | | | 28,0 | | 9,0 |

EP 0 299 873 B1

EP 0 299 873 B1

TABLEAU I (suite)

| Exemple n° | Conditions Expérimentales | Temps (h) | Teneur en % molaire produit | | | | | | |
|---|---|---|---|---|---|---|---|---|---|
| | | | $CH_3OH$ | $CH_3CHO$ | $HCOOCH_3$ | $C_2H_5OH$ | $CH_3COCH_3$ | AcOMe | Butanal |
| 12 | T = 180°C - $P_{(CO)}$ = 5MPa<br>$CH_3PPh_3I$ = 1 mmole<br>$HCO_2CH_3$ = 52 mmoles<br>$RhCl_2.3H_2O$ = 0,125 mmole | 2 | 5,8 | 25,0 | 58,4 | - | 10 | 0,8 | - |
| 13 | $PPN^+I^{-*}$ = 1 mmole<br>conditions<br>identiques au précédent | 2 | 6 | 31 | 57,1 | - | 3,9 | 2 | - |
| 14 | T = 184°C - $P_{(CO)}$=7 MPa<br>$CH_3N(Et)_3I$ = 2,5 mmoles<br>$HCOOCH_3$ = 167 mmoles<br>$RhCl_3. 3H_3O$=0,125 mmole<br>NMP = 50 $cm^3$ | 1 | 3,5 | 24,8 | 70,5 | - | 1,2 | - | - |
| 15 | T = 180°C - PCO = 10MPa<br>LiI =1 mmole<br>$RhCl_3.3H_2O$ = 0,125 mmole<br>Diméthyl imidazolidinone<br>= 50 $cm^3$ | 4 | 15,4 | 51,8 | 30 | - | 2,8 | - | - |
| 16 | T = 180°C. $P_{(CO)}$ =10 MPa<br>LiI = 1,1 mmole<br>$HCOOCH_3$ = 180 mmoles<br>$RhCl_3.3H_2O$ = 0,125 mmole<br>N- éthyl pyrrolidone<br>= 20 $cm^3$ | 1 | 9,1 | 16,1 | 74 | - | 0,8 | - | - |

TABLEAU I (suite)

| Exemple n° | Conditions Expérimentales | Temps (h) | Teneur en % molaire produit | | | | | | |
|---|---|---|---|---|---|---|---|---|---|
| | | | $CH_3OH$ | $CH_3CHO$ | $HCOOCH_3$ | $C_2H_5OH$ | $CH_3COCH_3$ | AcOMe | Butanal |
| 17 | T = 180°C. $P_{(CO)}$ = 7 MPa<br>LiI = 1,8 mmole<br>$HCOOH_3$ = 135 mmoles<br>IR ClCO($PPh_3$) = 0,125 mmole<br>NMP = 20 $cm^3$ | 1 | 10 | 23,5 | 66,5 | - | - | - | - |

\* $PPN^+I^-$ iodure de bis (triphényl phosporanylidène) ammonium

L'exemple comparatif 1 met en évidence le caractère déterminant du solvant.

Par ailleurs, les essais effectués en utilisant HI comme promoteur ont montré qu'en l'absence de $PPh_3$ - (Ex. comparatif 3, 4 et 5) l'activité est très faible alors qu'elle est importante en présence de $PPh_3$ (Exemple 11). Ces essais montrent également que sans $PPh_3$ et avec de fortes teneurs en HI (Exemple comparatif 5) on obtient essentiellement de l'acide acétique (comme l'indique DE-C-20 26 031).

Enfin l'exemple comparatif 6 montre qu'à une faible teneur en N-méthylpyrrolidone on obtient principalement de l'acétate de méthyle et de l'acide acétique et non pas de l'acétaldéhyde.

Exemple 18 :

On a opéré en utilisant le même protocole expérimental qu'à l'exemple 1, en utilisant les conditions suivantes : formiate de méthyle = 5 cm³ (87 mmoles), Rh = 0,12 mmole, LI = 3 mmoles, Pco = 8MPa, temps de réaction : 3 heures.

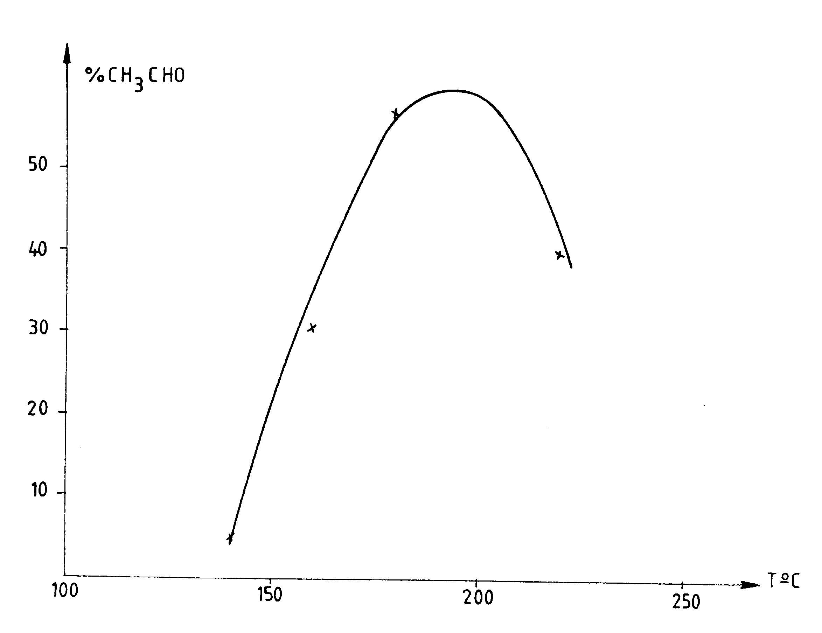

On a étudié la teneur en % molaire en acétaldéhyde en fonction de la température. Les résultats sont reportés sur la Fig. 1. Cette figure met en évidence l'effet déterminant de la température et le fait qu'une température d'environ 180°C permet d'obtenir le taux de transformation le plus grand en acétaldéhyde.

Dans ces mêmes conditions, la réaction fournit à plus haute température du butanal comme produit secondaire par réaction de condensation de l'acétaldéhyde (cf. Tableau II).

<u>TABLEAU II</u>

Influence de la température-sur le taux de transformation global (TTG) et la sélectivité.

| Temps (heure) | T° (°C) | TTG (%) | Sélectivité (%) | | |
|---|---|---|---|---|---|
| | | | $CH_3CHO$ | $CH_3OH$ | $C_4H_9OH$ (butanal) |
| 1 | 160 | 13,5 | 70 | 27 | - |
| | 180 | 30,5 | 78 | 16 | - |
| | 220 | 90 | 56 | 24 | 6 |
| 3 | 140 | 4 | 67 | 30 | - |
| | 160 | 32 | 88 | 9 | - |
| | 180 | 75 | 82 | 9 | 1,5 |
| | 220 | 98 | 43 | 24 | 16 |

<u>Conditions</u> : PCO = 5 MPa ; LiI = 1,9 mmoles ; RhCl₃, 3H₂O = 0,125 mmole, HCOOCH₃ = 87 mmoles, NMP = 50 ml

**Revendications**

1. Procédé de préparation d'un aldéhyde de formule

R - CHO

dans laquelle R est un groupe alkyle en C₁-C₇, caractérisé en ce que l'on fait réagir sur un formiate d'alkyle de formule

H - COOR

dans laquelle R à la signification donnée ci-dessus, un gaz constitué principalement de CO, en

10

présence de

a) un catalyseur à base d'un métal choisi parmi Rh, Ru, Ir, leur mélange ou un mélange de Rh et/ou de Ru et/ou de Ir avec Co, Ni ou Fe, ce catalyseur étant utilisé en une proportion molaire par rapport au formiate d'alkyle de $10^{-2}$ à $10^{-4}$,

b) un promoteur choisi parmi les iodures alcalins, alcalino-terreux, d'ammonium quaternaire ou de phosphonium, et les mélnages de composés covalents d'iode et d'une phosphine de formule $PR'_3$, $R'$ étant un groupe alkyle ou aryle ou d'une amine tertiaire de formule $NR''_3$, $R''$ étant un groupe alkyle, l'iodure ou le composé covalent d'iode étant utilisés en une proportion molaire par rapport au formiate d'alkyle de $4.10^{-2}$ à $0,25.10^{-2}$,

c) un solvant comprenant un N-(alkyl en $C_1$-$C_6$) amide cyclique, l'amide cyclique étant utilisé en une proportion molaire par rapport au formiate d'alkyle d'au moins 1.

2. Procédé selon la revendication 1, caractérisé en ce que l'on opère à une température de 150 à 230°C.

3. Procédé selon la revendication 2, caractérisé en ce que l'on opère à une température de 160 à 200°C.

4. Procédé selon l'une quelconque des revendications 1 à 3, caractérisé en ce que l'on opère à une pression de CO d'au moins 1 MPa.

5. Procédé selon la revendication 4, caractérisé en ce que l'on opère à une pression de CO d'au moins 5 MPa.

6. Procédé selon l'une quelconque des revendications 1 à 5, caractérisé en ce que le catalyseur est à base d'un métal choisi parmi Rh, Ru, Ir et leur mélange.

7. Procédé selon l'une quelconque des revendications 1 à 6, caractérisé en ce que le promoteur est constitué par un iodure alcalin, alcalino-terreux, d'ammonium quaternaire ou de phosphonium.

8. Procédé selon l'une quelconque des revendications 1 à 6, caractérisé en ce que le promoteur est constitué par un mélange d'iodure alcalin et de chrome hexacarbonyle.

9. Procédé selon l'une quelconque des revendications 1 à 6, caractérisé en ce que le promoteur est constitué par un mélange d'un composé covalent d'iode choisi parmi l'iode ou un iodure d'alkyle en $C_1$-$C_4$ ou l'acide iodhydrique avec une phosphine ou une amine tertiaire.

10. Procédé selon la revendication 9, caractérisée en ce que la teneur molaire en triphénylphosphine est au moins égale à la teneur molaire en composé covalent d'iode.

11. Procédé selon l'une quelconque des revendications 1 à 10, caractérisé en ce que le gaz constitué principalement de CO est un gaz d'aciérie.

**Claims**

1. Process for the preparation of an aldehyde of the formula

R - CHO

in which R is a $C_1$-$C_7$ alkyl group, characterised in that an alkyl formate of the formula

H - COOR

in which R has the above given meaning and, a gas constituted principally by CO are reacted in the presence of

a) a catalyst based on a metal selected from Rh, Ru, Ir, a mixture thereof or a mixture of Rh and/or of Ru and/or of Ir with Co, Ni or Fe, this catalyst being utilised in a molar proportion with reference to the alkyl formate of $10^{-2}$ to $10^{-4}$,

b) a promoter selected from alkali, alkaline earth, quaternary ammonium or phosphonium iodides and mixtures of covalent compounds of iodine and a phosphine of formula PR'$_3$, R' being an alkyl or

11

aryl group, or a tertiary amine of formula NR"$_3$, R" being an alkyl group, the iodide or the covalent compound of iodine being utilised in a molar proportion with respect to the alkyl formate of $4.10^{-2}$ to $0.25.10^{-2}$,

c) a solvent comprising an N-($C_1$-$C_6$ alkyl) cyclic amide, the cyclic amide being used in a molar proportion with respect to the alkyl formate of at least 1.

2. Process according to Claim 1, characterised in that it is carried out at a temperature of 150 to 230°C.

3. Process according to Claim 2, characterised in that it is carried out at a temperature of 160 to 200°C.

4. Process according to any one of Claims 1 to 3, characterised in that it is carried out at a CO pressure of at least 1 MPa.

5. Process according to Claim 4, characterised in that it is carried out at a CO pressure of at least 5 MPa.

6. Process according to any one of Claims 1 to 5, characterised in that the catalyst is based on a metal chosen from Rh, Ru, Ir and a mixture thereof.

7. Process according to any one of Claims 1 to 5, characterised in that the promoter is constituted by an alkaline, alkaline earth, quaternary ammonium or phosphonium iodide.

8. Process according to any one of Claims 1 to 6, characterised in that the promoter is constituted by a mixture of alkali iodide and chromium hexacarbonyl.

9. Process according to any one of Claims 1 to 6, characterised in that the promoter is constituted by a mixture of a covalent compound of iodine selected from iodine or a $C_1$-$C_4$ alkyl iodide or hydroiodic acid with a phosphine or a tertiary amine.

10. Process according to Claim 9, characterised in that the molar content of triphenylphosphine is at least equal to the molar content of covalent iodine compound.

11. Process according to any one of Claims 1 to 10, characterised in that the gas constituted principally by CO is a gas from steelworks.

**Patentansprüche**

1. Verfahren zur Herstellung eines Aldehyds der Formel

R-CHO ,

worin R eine $C_1$-$C_7$-Alkyl-Gruppe ist, dadurch gekennzeichnet, daß ein Alkylformiat der Formel

H-COOR ,

worin R die vorstehend gegebene Bedeutung hat, umgesetzt wird mit einem Gas, das hauptsächlich aus CO besteht, in Gegenwart

a) eines Katalysators auf der Grundlage eines Metalls, ausgewählt aus Rh, Ru, Ir, deren Mischung oder einer Mischung aus Rh und/oder Ru und/oder Ir mit Co, Ni oder Fe, wobei der Katalysator in einem Molverhältnis zum Alkylformiat von $10^{-2}$ bis $10^{-4}$ eingesetzt wird,

b) eines Promotors, ausgewählt aus Alkali-, Erdalkali-, quartären Ammonium- oder Phosphoniumiodiden und Mischungen von covalenten Iod-Verbindungen und einem Phosphin der Formel PR'$_3$, worin R' eine Alkyl- oder Aryl-Gruppe ist, oder einem tertiären Amin der Formel NR"$_3$, worin R" eine Alkyl-Gruppe ist, wobei das Iodid oder die covalente Iod-Verbindung in einem Molverhältnis zum Alkylformiat von $4 \cdot 10^{-2}$ bis $0,25 \cdot 10^{-4}$ eingesetzt werden,

c) einem Lösungsmittel, umfassend ein cyclisches N-($C_1$-$C_6$-Alkyl)amid, wobei das cyclische Amid in einem Molverhältnis zum Alkylformiat von wenigstens 1 eingesetzt wird.

2. Verfahren nach Anspruch 1, dadurch gekennzeichnet, daß bei einer Temperatur von 150 bis 230°C gearbeitet wird.

3. Verfahren nach Anspruch 2, dadurch gekennzeichnet, daß bei einer Temperatur von 160 bis 200°C gearbeitet wird.

4. Verfahren nach irgendeinem der Ansprüche 1 bis 3, dadurch gekennzeichnet, daß bei einem CO-Druck von wenigstens 1 MPa gearbeitet wird.

5. Verfahren nach Anspruch 4, dadurch gekennzeichnet, daß bei einem CO-Druck von wenigstens 5 MPa gearbeitet wird.

6. Verfahren nach irgendeinem der Ansprüche 1 bis 5, dadurch gekennzeichnet, daß der Katalysator auf einem Metall basiert, ausgewählt aus Rh, Ru, Ir und deren Mischungen.

7. Verfahren nach irgendeinem der Ansprüche 1 bis 6, dadurch gekennzeichnet, daß der Promotor aus einem Alkali-, Erdalkali-, quartären Ammonium- oder Phosphoniumiodid besteht.

8. Verfahren nach irgendeinem der Ansprüche 1 bis 6, dadurch gekennzeichnet, daß der Promotor aus einer Mischung eines Alkaliiodids und Chromhexacarbonyl besteht.

9. Verfahren nach irgendeinem der Ansprüche 1 bis 6, dadurch gekennzeichnet, daß der Promotor aus einer Mischung einer covalenten Iod-Verbindung, ausgewählt aus Iod oder einem $C_1$-$C_4$-Alkyliodid oder Iodwasserstoffsäure, mit einem Phosphin oder einem tertiären Amin besteht.

10. Verfahren nach Anspruch 9, dadurch gekennzeichnet, daß der Molgehalt an Triphenylphosphin wenigstens gleich dem Molgehalt an covalenter Iod-Verbindung ist.

11. Verfahren nach irgendeinem der Ansprüche 1 bis 10, dadurch gekennzeichnet, daß das hauptsächlich aus CO bestehende Gas ein Stahlhüttengas ist.